Europäisches Patentamt

European Patent Office

Office européen des brevets

(19)

(11) Veröffentlichungsnummer: **0 263 417**

**A1**

(12) **EUROPÄISCHE PATENTANMELDUNG**

(21) Anmeldenummer: 87114207.1

(22) Anmeldetag: 29.09.87

(51) Int. Cl.⁴: **A61K 37/54**

(30) Priorität: 30.09.86 AT 2603/86

(43) Veröffentlichungstag der Anmeldung:
13.04.88 Patentblatt 88/15

(84) Benannte Vertragsstaaten:
AT BE CH DE ES FR GB GR IT LI LU NL SE

(71) Anmelder: BIOCHEMIE Gesellschaft m.b.H.

A-6250 Kundl Tirol(AT)

(72) Erfinder: Baumgartner, Gerhard
Magaretengürtel 4
A-1050 Wien(AT)
Erfinder: Baumgartner, Marga
Magaretengürtel 4
A-1050 Wien(AT)
Erfinder: Horaczek, Alfred
Gentzgasse 10/5/11
A-1180 Wien(AT)

(74) Vertreter: Norris, Allen Edwin et al
SANDOZ AG Patentabteilung Lichtstrasse 35
CH-4002 Basel(CH)

(54) **Verwendung von Hyaluronidase.**

(57) Verwendung von Hyaluronidase und Hyaluronidase enthaltender Präparate, gegebenenfalls in Kombination mit Zytostatika, zur Behandlung von Gehirntumoren (Gliomen).

EP 0 263 417 A1

## Verwendung von Hyaluronidase

Bei der Behandlung primärer und sekundärer Hirntumoren sieht man sich mit besonderen Schwierigkeiten konfrontiert, die eine Verbesserung der Behandlungsaussichten, wie sie bei anderen Tumorlokalisationen in den letzten Jahren erzielt werden konnte, bisher verhindert haben. Bei Gliomen ist einerseits eine radikale chirurgische Sanierung nicht möglich, andererseits hat auch die Strahlen-und die Chemotherapie, sowie deren Kombination, bisher keine Besserung der Prognose bewirken können. Besondere Schwierigkeiten in der Behandlung der Gliome sind die schlechte Abgrenzbarkeit der Tumoren, die zytologische und histologische Heterogenität mit unterschiedlicher Chemosensibilität der Tumorzellen und weiters die Blut-Hirnschranke als Hemmung des Antransportes der Zytostatika zum Tumor. Die mit diesen Problemen möglicherweise im Zusammenhang stehende geringe Chemosensibilität der Gliome war Anlaß nach Auswegen zu suchen, wobei bisher versucht wurde, durch Öffnung der Blut-Hirnschranke, z.B. mit Mannit eine Besserung des Antransportes und dadurch auch der Wirkung der Chemotherapeutika zu erzielen, es konnte jedoch auch mit dieser Methode in der Mehrzahl der Fälle keine Steigerung der Wirkung der Chemotherapie erzielt werden.

Der Grund liegt vermutlich in der Tatsache, daß die Blut-Hirnschranke bei Tumoren häufig bereits gestört ist, weiters bei multifokalen cerebralen Tumormanifestationen die Blut-Hirnschrankenöffnung in einem bestimmten Gefäßgebiet nur einen Teil der Tumoren erfaßt und letztlich auch in der Tatsache, daß eine Anhebung des Zytostatikaspiegels im Liquor keineswegs immer eine ausreichende Anhebung des Zytostatikaspiegels im übrigen Hirnparenchym und insbesondere im Tumor entsprechen muß.

Hyaluronidase, ein Ferment mit einem Molekulargewicht von 61 000, das Hyaluronsäure und Chondroitinsulfat, wichtige Bestandteile der Grundsubstanz des Bindegewebes, spaltet, spielt im Bindegewebsstoffwechsel und auch bei der Reifung und Differenzierung embryonaler Gewebe, z.B. bei der Knochenentstehung, eine Rolle. Therapeutisch wurde Hyaluronidase bisher in der Behandlung des Hirnoedems, des akuten Stadium des Herzinfarktes und als Zusatz zu in größeren Flüssigkeitsmengen gelösten Medikamenten bei subkutaner Applikation bzw. in der Lokaltherapie von Pleuraergüssen eingesetzt. Mit Hyaluronidase kann daher auch eine Beschleunigung der Resorption von paravenös gespritzten Zytostatika erzielt und eine lokale Nekrosebildung in den meisten Fällen verhindert werden.

Überraschenderweise wurde nun gefunden, daß die Verabreichung von Hyaluronidase, vorzugsweise in Kombination mit Cytostatika, zu einer wesentlichen Verbesserung bei der Behandlung von Gliomen führt.

Hyaluronidase ist ein Enzym, das aus verschiedenen Rohstoffquellen gewonnen werden kann. Es baut Hyaluronsäure, den Hauptbestandteil der interzellulären Kittsubstanz, ab und bewirkt dadurch eine gesteigerte Diffusion der extrazellulären Körperflüssigkeit, wie auch von außen zugeführter Flüssigkeit. So findet Hyaluronidase aus Rindertestes seit vielen Jahren therapeutische Anwendung zur subkutanen Infusion von hochmolekularen Infusionsflüssigkeiten, zur Verflüssigung viskoser Ergüsse in Gelenken und Schleimbeuteln oder zur Resorptionsbeschleunigung von intramskulären oder subkutanen Injektionen. Diese Anwendungen sind nur als Beispiele zur werten. Meistens wird die Wirksamkeit von Hyaluronidasepräparaten in internationalen Einheiten (I.E.), verglichen mit einem internationalen Standard, gemessen. Je nach Aufwand können Hyaluronidasepräparate verschiedener Stärke hergestellt werden, d. h., die pro mg Wirksubstanz nachweisbaren Aktivitätseinheiten können stark differieren. Naturgemäß ist man bestrebt, den Wirkstoff Hyaluronidase, ein Glycoprotein, in möglichst hoher Reinheit, z.B. 50.000 I.E./mg und mehr herzustellen. Man muß dabei aber geringere Ausbeuten und eine verringerte Stabilität in Kauf nehmen. Für die oben genannten Indikationen haben sich spezifische Aktivitäten von 1000-2000 I.E./mg als ausreichend erwiesen. Für die intravenöse Applikation, z.B. nach Herzinfarkt, strebt man wesentlich höhere Werte, nämlich etwa 50.000 I.E./mg an.

Als Reinigungsschritte, die sich an die Extraktion der tierischen Drüsen anschließen, werden Methoden verwendet, wie sie der Herstellung von Wirkstoffen aus organischem Material üblich sind. Hiezu zählen Fällungen mit anorganischen Salzen, chromatographische Reinigungsverfahren an verschiedenen Trägern und Entsalzung. Die Endprodukte werden schließlich in lyophilisierter Form, allenfalls unter Zusatz von Stabilisatoren, isoliert.

Ein für diesen Zweck geeignetes Präparat ist z.B. Permease, eine Hyaluronidasebereitung im Ampullen mit je 750 I.E. lyophilisierter Trockensubstanz pro Ampulle (Firma Sanabo). Die Hyaluronidase kann in den Zubereitungen mit Lösungsmittel und/oder Stabilisierungsmittel, z.B. mit Mannit oder hydrolysierter Gelatine vermischt sein.

Andere geeignete Präparate sind beispielsweise Alidase, Diffusin, Enzodase, Hyazyme, Wydase, Kinaden, Adose u.a.m.

Die in diesen Präparaten enthaltene Hyaluronidase kann beispielsweise folgendermaßen erhalten werden:

Rindertestes werden tiefgefroren zerkleinert und mit verdünnter Essigsäure über Nacht extrahiert. Nach der Entfernung der festen Anteile wird nach Zusatz von Ammonsulfat mit einer Kammerzentrifuge geklärt, abermals Ammonsulfat zugesetzt und der Niederschlag isoliert. Man löst in Wasser, klärt durch Filtration und entsalzt mit Sephadex G 25. Aus der Lösung wird mit Bleiacetat eine Fällung erzeugt, die abgetrennt und im - schwach sauren Bereich gelöst wird. Nach dem Blankfiltrieren wird wie vorher entsalzt, ehe mit DEAE-Sephadex behandelt und mit Ammonsulfat gefällt wird. Der zentrifugierte Niederschlag wird wieder gelöst, über Sephadex G 25 entsalzt, die Aktivität bestimmt und soviel inertes Verdünnungsmittel, z.B. Mannit oder Lactose zugegeben, daß nach dem Lyophilisieren eine spezifische Aktivität des Endproduktes von 1000 - 2000 I.E./mg resultiert.

Für die erfindungsgemäße Verwendung sind auch Präparate geeignet, die Hyaluronidase in größerer Reinheit und Konzentration enthalten, z.B. 5.000 bis 350.000 I.E. Die Herstellung solcher Hyaluronidasepräparate wird beispielsweise in der DE-OS 3,148,517, in Yak. zasshi 100(8)/832-838(1980) u.a. beschrieben. Ein solches Präparat kann beispielsweise folgendermaßen erhalten werden:

Rindertestes werden tiefgefroren zerkleinert und mit verdünnter Essigsäure über Nacht extrahiert. Nach dem Entfernen der festen Anteile über einen Dekanter wird blankfiltriert und in CM-Sephadex eingetragen (pH = 4,8 - 5,0). Am nächsten Tag wird der Ionenaustauscher isoliert, gründlich bei pH = 5,0 gewaschen, anschließend mit Acetatpuffer (pH = 5,5) eluiert und das Eluat an Sephadex G 25 entsalzt. Nach dem Lyophilisieren erhält man ein Produkt der spezifischen Aktivität von etwa 2000 - 5000 I.E./mg. Dieses Produkt wird in Acetatpuffer (pH = 5) gelöst und über CM-Sepharose 6 BCL filtriert. Nach dem Nachwaschen wird mit einem Acetatpuffer (pH = 5,5) eluiert, die Fraktionen des Ablaufes zwischen pH = 5,1 und 5,5 gesammelt und wieder mit Sephadex G 25 entsalzt und lyophilisiert. Man erhält ein Produkt mit 20.000 - 40.000 I.E./mg. Dieses Produkt kann bei pH = 7,5 in Phosphatpuffer gelöst und mit DEAE-Sephadex bei 4°C gerührt werden. Nach Entfernen des Ionenaustauschers wird mit Essigsäure auf pH = 5,0 gestellt, über Sephadex G 25 entsalzt und lyophilisiert. Die Aktivität des Endproduktes liegt bei 40.000 - 60.000 I.E./mg.

Für die erfindungsgemäße Verwendung der Hyaluronidase werden Präparate mit einem Gehalt von 5.000 bis 300.000 I.E., insbesondere von 7.500 bis 200.000 I.E. bevorzugt. Die Verabreichung kann i.m. oder i.v. erfolgen, wobei bei höheren Dosen die i.v.-Verabreichung (per Infusion) bevorzugt ist.

Die erfindungsgemäße Wirkung von Hyaluronidase wurde zum Beispiel in einer Studie bei bisher 17 Patienten mit Gliomen nachgewiesen. 100.000 bzw. 200.000 I.E. Hyaluronidase wurden jeweils in 100 ml physiologischer Kochsalzlösung gelöst und intravenös verabreicht. Am Tag 1 wurden 80 mg/m$^2$ CCNU oral gegeben, anschließend 100.000 I.E. Hyaluronidase, anschließend 120 mg Methotrexat/m$^2$ ebenfalls gelöst in 100 mg physiologischer Kochsalzlösung, 4 Stunden später wurden neuerlich 100.000 I.E. Hyaluronidase, gelöst in 100 ml physiologischer Kochsalzlösung, weiters 60 mg/m$^2$ Etoposid, gelöst in 100 ml physiologischer Kochsalzlösung, 750/m$^2$ Fluoruracil, gelöst in 250 ml 5%iger Dextrose. Über den Tag verteilt wurden 3x20 m Val Natriumbikarbonat verabreicht. Am Tag 2 und Tag 3 wurden jeweils 500 ml Laevulose, 500 ml 10%iges Mannit, 200.000 I.E. Hyaluronidase, gelöst in 100 ml physiologischer Kochsalzlösung, 25 mg/m$^2$ Cis-Platin in 20 ml physiologischer Kochsalzlögung, 60 mg/m$^2$ Etoposid, gelöst in 100 ml physiologischer Kochsalzlösung und 3x20 Val Natriumbikarbonat verabreicht. Am Tag 3 wurden zusätzlich 180 mg Calciumfolinat, gelöst in 250 mg physiologischer Kochsalzlösung als Antidot zum Methotrexat verabreicht.

Die Therapie wurde im allgemeinen sehr gut vertragen. Es traten Nebenwirkungen auf, die nach den verabreichten Zytostatika zur erwarten waren. Hydaluronidase selbst führte zu keinerlei Nebenwirkungen.

Computeromographisch verifizierte Teilremissionen wurden in 9 von 17 Fällen erzielt, in 4 Fällen blieb die Tumorgrösse unverändert, in einem Fall blieb der Tumor progredient. Computertomographie-Kontrollen fehlten vorerst in 3 Fällen. Der neurologische Zustand wurde in 7 Fällen gebessert, bei 8 Patienten blieb er unverändert, und bei 2 Patienten verschlechterte er sich. Die Beobachtunsdauer der Patienten beträgt zwischen 1 und 12 Monaten. 3 Patientinnen starben, eine an Hirnblutung, eine weitere an Lungenembolie im Therapieintervall und eine an einem Rezidiv nach durch 9 Monate bestehender Remission.

**Ansprüche**

1. Verwendung von Hyaluronidase oder Hyaluronidase enthaltender Präparate zur Behandlung von Gehirntumoren (Gliomen).

2. Verwendung von Hyaluronidase oder Hyaluronidase enthaltender Präparate in Kombination mit Zytostatika zur Behandlung von Gehirntumoren (Gliomen).

3. Verwendung von Hyaluronidase oder Hyaluronidase enthaltender Präparate zur Herstellung eines Medikamentes zur Behandlung von Gehirntumoren (Gliomen).

4. Verwendung von Hyaluronidase oder Hyaluronidase enthaltender Präparate zur Herstellung eines Medikaments zur Behandlung, in Kombination mit Zytostatika, von Gehirntumoren (Gliomen).

5. Verfahren zur Behandlung von Gehirntumoren in Patienten die eine solche Behandlung bedürfen, bestehend darin, dass man Hyaluronidase oder Hyaluronidase enthaltende Präparate in einer für diesen Zweck effektive Menge verabreicht.

Europäisches Patentamt

**EUROPÄISCHER TEILRECHERCHENBERICHT,**
der nach Regel 45 des Europäischen Patentübereinkommens für das weitere Verfahren als
europäischer Recherchenbericht gilt

Nummer der Anmeldung

EP 87 11 4207

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CHEMICAL ABSTRACTS, Band 78, Nr. 12, 26. März 1973, Seite 91, Zusammenfassung Nr. 80359x, Columbus, Ohio, US; D. SENITZ et al.: "Intravenous application of hylase Dessau. Experimental studes in rats. III. Effects of hylase Dessau on the blood-brain barrier", & EXP.PATHOL. 1972, 7(5-6), 228-33 | | A 61 K 37/54 |
| | * Zusammenfassung * | 3,4 | |
| | -- | | |
| X | FR - M - 7734 (BIOREX) | | |
| | * Seite 2, Zeilen 29-34 * | 3,4 | |
| | -- | | |
| X | EP - A - 0 193.330 (BIOPHARM UK LTD) | | |
| | * Seite 8, Zeilen 7-12 * | 3,4 | |
| | -- | | |
| A | GB - A - 651 545 (ORTHO PHARMACEUTICAL CO.) | | RECHERCHIERTE SACHGEBIETE (Int. Cl.4) |
| A | DE - A - 2 440 529 (THE GREEN CROSS CORP.) | | |
| | ---------------------------------------- | | A 61 K |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich
ist, auf der Grundlage einiger Patentansprüche sinnvolle Ermittlungen über den Stand der Technik
durchzuführen.

Vollständig recherchierte Patentansprüche: 3,4

Unvollständig recherchierte Patentansprüche:

Nicht recherchierte Patentansprüche: 1,2,5

Grund für die Beschränkung der Recherche:

Verfahren zur chirurgischen oder
therapeutischen Behandlung des
menschlichen oder tierischen Körpers
(siehe Art. 52(4) des Europäischen
Patentübereinkommens).

| Recherchenort | Abschlußdatum der Recherche | Prüfer |
|---|---|---|
| Den Haag | 10-12-1987 | SKELLY |